(19) Europäisches Patentamt
European Patent Office
Office européen des brevets

(11) Veröffentlichungsnummer: **0 149 814**
**B1**

(12) **EUROPÄISCHE PATENTSCHRIFT**

(45) Veröffentlichungstag der Patentschrift: **27.04.88**

(51) Int. Cl.⁴: **C 07 D 405/06**, A 61 K 31/41, A 61 K 31/415

(21) Anmeldenummer: **84115718.3**

(22) Anmeldetag: **18.12.84**

# (54) Azolylmethylcycloacetale, Verfahren zu ihrer Herstellung und ihre Verwendung als Arzneimittel.

(30) Priorität: **19.01.84 DE 3401694**

(43) Veröffentlichungstag der Anmeldung: **31.07.85 Patentblatt 85/31**

(45) Bekanntmachung des Hinweises auf die Patenterteilung: **27.04.88 Patentblatt 88/17**

(84) Benannte Vertragsstaaten: **AT BE CH DE FR GB IT LI NL**

(56) Entgegenhaltungen:
**EP-A-0 049 565**
**DE-A-2 804 096**
**US-A-4 375 474**

(73) Patentinhaber: **BASF Aktiengesellschaft, Carl-Bosch- Strasse 38, D-6700 Ludwigshafen (DE)**

(72) Erfinder: **Janssen, Bernd, Dr., Leuschnerstrasse 18a, 6700 Ludwigshafen (DE)**
Erfinder: **Kohlmann, Friedrich- Wilhelm, Dr., An der Duene 6, 2082 Moorrege (DE)**
Erfinder: **Wesenberg, Walter, Dorfstrasse 16, 2421 Bujendorf ueber Eutin (DE)**
Erfinder: **Heberle, Wolfgang, Dr., Gebelsbergstrasse 41, 7000 Stuttgart 1 (DE)**

**EP 0 149 814 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue Azolylmethylcycloacetale, Verfahren zu deren Herstellung und diese enthaltende therapeutische Mittel, die als antimykotika verwendet werden können, sowie deren Verwendung bei der Heilung von Krankheiten.

Es sind bereits zahlreiche antimykotische Wirkstoffe, z. B. Azolylmethylcarbinole wie Miconazol (DE-OS 19 40 388) oder auch Azolylmethyldioxolane wie Ketoconazol (DE-OS 28 04 096) bekammt. Ihre Wirkungen befriedigen jedoch nicht immer (Zeitschrift für Hautkrankheiten 56, 1109 (1981)). Von den Verbindungen des letztgenannten Typs unterscheiden sich die Verbindungen der vorliegenden Erfindung wesentlich durch die Art der Substituenten vor allem in 5- bzw. 4-Stellung des Dioxan- bzw. Dioxolan-Gerüstes.

Es wurde nun gefunden, daß Azolylmethylcycloacetale der Formel I

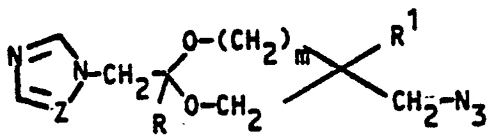 (I),

in der

R ein Phenylrest, der durch Halogen substituiert sein kann, oder $C_{1-6}$-Alkylgruppe,
$R^1$ Wasserstoff oder ein $C_{1-3}$-Alkylrest sein kann,
Z CH oder N und
m die Zahl 0 oder 1

bedeuten, sowie deren physiologisch verträgliche Säureadditionssalze gute antimikrobielle, insbesondere antimykotische Eigenschaften aufweisen.

In der Formel I stellt R vorzugsweise einen tert.-Butylrest oder einen durch Halogen, insbesondere Chlor, substituierten Phenylrest dar. Als letzterer ist die 2,4-Dichlorphenylgruppe besonders geeignet.

$R^1$ ist vorzugsweise Wasserstoff oder eine Methylgruppe.

Die neuen Verbindungen der Formel I enthalten chirale Zentren und werden im allgemeinen in Form von Racematen oder als Diastereomeren-Gemische von erythro- sowie threo-Formen erhalten. Die erythro- und threo-Diastereomeren lassen sich bei den erfindungsgemäßen Verbindungen beispielsweise aufgrund ihrer unterschiedlichen Löslichkeit oder durch Säulenchromatographie trennen und in reiner Form isolieren. Aus solchen einheitlichen Diastereomerenpaaren kann man mit bekannten Methoden einheitliche Enantiomere erhalten. Als antimikrobielle Mittel kann man sowohl die einheitlichen Diastereomeren bzw. Enantiomeren wie auch deren Gemische verwenden.

Als Säuren zur Bildung physiologisch verträglicher Salze kommen vorzugsweise Halogenwasserstoffsäuren, wie Bromwasserstoffsäure und insbesondere Chlorwasserstoffsäure, mit der die erfindungsgemäßen Verbindungen besonders gut kristallisierende Salze bilden, in Frage. Ferner sind zu nennen: Phosphorsäure, Salpetersäure, Schwefelsäure, mono- und bifunktionelle Carbonsäuren und Hydroxycarbonsäuren - wie Essigsäure, Oxalsäure, Maleinsäure, Bernsteinsäure, Fumarsäure, Weinsäure, Zitronensäure, Salicylsäure, Sorbinsäure, Milchsäure -, sowie Sulfonsäuren - wie p-Toluolsulfonsäure und 1,5-Naphthalindisulfonsäure -.

Die neuen Azolylmethylcycloacetale der Formel I lassen sich herstellen, indem man

a) ein Cycloacetal der Formel II

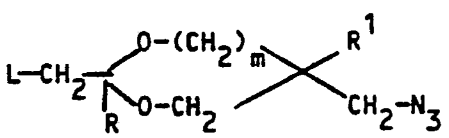 (II),

in der R, $R^1$ und m die oben angegebene Bedeutung haben und L eine nucleophil substituierbare Abgangsgruppe darstellt, mit einer Verbindung der Formel III

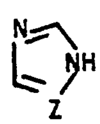 (III),

in der Z die oben angegebene Bedeutung hat, oder

b) bein Cycloacetal der Formel IV

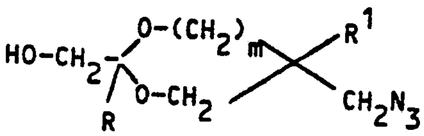

(IV),

worin R, $R^1$ und m die oben angegebene Bedeutung besitzen, mit einer Verbindung der Formel V

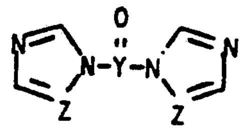

(V),

in welcher Z die oben angegebene Bedeutung hat und Y ein Kohlenstoff- oder Schwefelatom bedeutet, oder

c) eine Verbindung der Formel VI

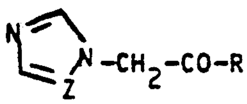

(VI),

in der Z und R die oben angegebene Bedeutung haben, mit einer Verbindung der Formel VII

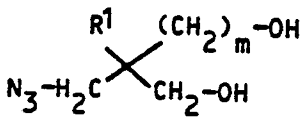

(VII),

in der $R^1$ und m die oben angegebene Bedeutung haben, oder

d) eine Verbindung der Formel VIII

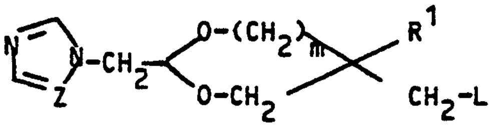

(VIII),

in der Z, R, $R^1$ und m die oben angegebene Bedeutung haben und L eine nucleophil substituierbare Abgangsgruppe darstellt, mit einem Alkalimetallazid

umsetzt und die so erhaltenen Verbindungen gegebenenfalls in ihre physiologisch verträglichen Säureadditionssalze überführt.

Von diesen Verfahren ist das letztere (d) bevorzugt.

Die Reaktion a) erfolgt vorzugsweise in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C. Zu den bevorzugten Lösungs- und Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind Alkalihydroxide wie Lithium-, Natrium- oder Kaliumhydroxid, Alkalicarbonate wie Natrium- oder Kaliumcarbonat oder Natrium- und Kaliumhydrogencarbonat, Pyridin, 4-Dimethylaminopyridin sowie Überschüsse an 1,2,4-Triazol oder Imidazol. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid, Benzyltriethylammoniumchlorid oder -bromid oder Kronenether wie 12-Krone-4, 15-Krone-5, 18-Krone-6, Dibenzo-18-Krone-6, Dicyclohexano-18-Krone-6 in Frage.

Die Umsetzung wird im allgemeinen bei Temperaturen zwischen 10 und 120°C drucklos oder unter Druck, kontinuierlich oder diskontinuierlich durchgeführt.

Die Reaktion a) kann auch so durchgeführt werden, daß man die Verbindung III in ein Metallsalz, vorzugsweise ein Alkalisalz überführt und dieses gegebenenfalls in Gegenwart eines Lösungs- oder Verdünnungsmittels und gegebenenfalls unter Zusatz einer starken anorganischen oder organischen Base bei Temperaturen zwischen -10 und +120°C mit der Verbindung II umsetzt. Zu den bevorzugten Lösungs- oder Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und Sulfolan.

Geeignete Basen, die auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium oder Kalium-tert.-butoxid, Lithium-, Natrium- oder Kalium-Triphenylmethyl und Naphthalin-Lithium, -Natrium oder -Kalium.

L ist vorzugsweise ein Halogenatom oder eine reaktive Estergruppe, z. B. die Mesylatgruppe.

Die Reaktion b) wird in einem Lösungs- oder Verdünnungsmittel durchgeführt. Als solche kommen polare organische Lösungsmittel wie Nitrile, z. B. Acetonitril, Sulfoxide, z. B. Dimethylsulfoxid, Formamide, z. B. Dimethylformamid, Ketone, z. B. Aceton, Ether, z. B. Diethylether, Tetrahydrofuran und insbesondere Chlorkohlenwasserstoffe, z. B. Methylenchlorid und Chloroform in Frage.

Man arbeitet im allgemeinen zwischen 0 und 100°C, vorzugsweise bei 20 bis 80°C. Bei Anwesenheit eines Lösungsmittels wird zweckmäßigerweise beim Siedepunkt des jeweiligen Lösungsmittels gearbeitet.

Bei der Durchführung des Verfahrens b) setzt man auf 1 Mol der Verbindung der Formel IV etwa 1 Mol Carbonyl-bis-1,2,4-triazol-(1) bzw. -imidazol-(1) oder vorzugsweise etwa 1 Mol Sulfonyl-bis-1,2,4-triazol-(1) bzw. -imidazol-(1) ein oder erzeugt Sulfonyl-bis-1,2,4-triazol-(1) bzw. -imidazol-(1) in situ.

Die Reaktion c) erfolgt vorzugsweise durch Erhitzen der beiden Reaktanten unter azeotroper Wasserabtrennung in einem geeigneten organischen Lösemittel unter Rückfluß, vorzugsweise in Gegenwart eines einfachen Alkohols, wie Ethanol, Propanol, Butanol oder Pentanol und in Gegenwart einer geeigneten starken Säure, wie 4-Methylbenzolsulfonsäure. Geeignete organische Lösemittel sind z. B. aromatische Kohlenwasserstoffe, wie Benzol, Methylbenzol und Dimethylbenzol sowie gesättigte Kohlenwasserstoffe, wie Cyclohexan oder entsprechende Gemische.

Die Reaktion d) erfolgt vorzugsweise in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C. Zu den bevorzugten Lösungs- und Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind beispielsweise Alkalihydroxide wie Lithium-, Natrium- oder Kaliumhydroxid, Alkalicarbonate wie Natrium- oder Kaliumcarbonat oder Natrium- und Kaliumhydrogencarbonat, Pyridin oder 4-Dimethylaminopyridin oder auch Überschüsse an Alkalimetallazid.

Als Reaktionsbeschleuniger kommen die für das Verfahren a) genannten Substanzen in Betracht.

Die Umsetzung wird im allgemeinen bei Temperaturen zwischen 10 und 120°C drucklos oder unter Druck, kontinuierlich oder diskontinuierlich durchgeführt.

Die so erhaltenen Verbindungen der Formel I werden nach üblichen Methoden isoliert und gegebenenfalls mit den genannten Säuren in ihre Salze überführt.

Die Ausgangsverbindungen II sind auf folgendem Weg zugänglich:

$$VII + L\text{-}CH_2\text{-}CO\text{-}R \xrightarrow[-H_2O]{H^+} II$$

(IX)

Dazu setzt man die Reaktanten entsprechend dem oben beschriebenen Verfahren b) unter azeotroper Wasserabscheidung um.

Die Ausgangsverbindungen der Formel VI sind bereits bekannt, bzw. können in an sich bekannter Weise hergestellt werden, beispielsweise gemäß DE-PS 25 49 798 oder DE-OS 29 40 133.

Die Ausgangsverbindungen VII sind gemäß folgendem Schema zugänglich:

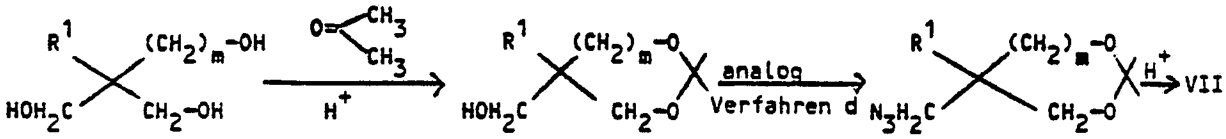


(X) (XI) (XII)

Die Ausgangsverbindungen der Struktur VIII sind teilweise literaturbekannt (J. Med. Chem. 12, 788 (1969) und 22, 1003 (1979), US-PSen 4,101,664; 4,101,665; 4,139,540; 4,338,327), bzw. können entsprechend hergestellt werden.

Überraschenderweise zeigen die erfindungsgemäßen Azolderivate neben einer sehr guten antimykotischen In-vitro-Wirksamkeit eine bessere, therapeutisch nutzbare In-vivo-Wirksamkeit als bekannte Präparate,

insbesondere gegen Dermatophyten, aber auch gegen Candida. Sie besitzen auch antibakterielle Wirksamkeiten. Die erfindungsgemäßen Wirkstoffe stellen somit eine wertvolle Bereicherung der Pharmazie dar.

Die Wirkung gegen Dermatophyten, Bakterien und Protozoen kann nach Methoden, wie sie beispielsweise in P. Klein, Bakteriologische Grundlagen der chemotherapeutischen Laboratoriumspraxis, Springer-Verlag Berlin, 1957, beschrieben wird, aufgezeigt werden. Die Wirkung gegenüber Hefen wurde im Pseudomycel- bzw. Mycelphasentest mit Candida albicans nachgewiesen (vgl. DE-OS 30 10 093).

Es wurde geprüft, welche minimalen Hemmkonzentrationen (MHK-Werte) im Agardilutionstest erreicht werden.

In der Tabelle 1 sind die Ergebnisse zusammengefaßt.

**Tabelle 1**

Antimikrobielle Wirksamkeit der neuen Verbindung in vitro

| Substanz des Bsp. Nr.: | Epid. flocc. | Microsp. ferrug. | Trichoph. ment. | Cand. alb. Hefe-phase | Cand. alb. Mycel-phase | Abs. corym. | Asp. fum. | Mucor pus. | Staph. aur. |
|---|---|---|---|---|---|---|---|---|---|
| 1 | 0,25 | 0,125 | 1 | >16 | 0,0625 | >16 | 4 | 8 | 128 |
| 2 | 0,0156 | 0,0156 | 0,0312 | >16 | 0,0625 | 4 | 1 | 1 | 128 |
| 7 | 0,125 | 0,0625 | 0,0125 | >16 | 0,0625 | 16 | 0,5 | 8 | 128 |
| Keto-conazol | 4 | 1 | 2 | >128 | 0,0312 | 32 | 128 | 16 | 128 |

Im Modell der Meerschweinchen-Trichophytie (Trichophyton mentagrophytes), vgl. Heffter-Heubner: Handbuch der exp. Pharmakologie, Vol. XVI/II A, sind die neuen Verbindungen bei äußerlicher Anwendung auch rezidivfrei besser wirksam als die mitgeprüfte Vergleichssubstanz.

Die Wirkung der Prüfsubstanzen bei topischer Anwendung im Modell der experimentellen Candida albicans-Vaginitis war ebenfalls gut.

Die neuen Verbindungen sind auch oral wirksam. Im Modell der experimentellen generalisierten Candidose der Maus bzw. im Modell der experimentellen Vaginitis mit Candida albicans an der Ratte konnten mit den Prüfsubstanzen in niedrigen therapeutischen Dosen nach oraler Gabe gute Ausheilungen der Infektionen erreicht werden.

Die neuen Verbindungen sind daher besonders zur äußerlichen, aber auch oralen Behandlung von Pilzinfektionen an Mensch und Tier geeignet. Als Indikationsgebiete an Mensch und Tier sind beispielsweise zu nennen: Dermatomykosen, insbesondere verursacht durch Dermatophyten, wie Spezies der Gattungen Epidermatophyton, Microsporum oder Trichophyton, Hefen, wie Spezies der Gattungen Candida und Schimmelpilze, wie Spezies der Gattungen Aspergillus, Mucor oder Absidia.

Die Verbindungen können allein oder zusammen mit anderen bekannten Wirkstoffen, insbesondere Antibiotika, verwendet werden.

Die Herstellung der chemotherapeutischen Mittel oder Zubereitungen, die mit üblichen festen, halbfesten oder flüssigen Trägerstoffen oder Verdünnungsmitteln und den üblicherweise verwendeten pharmazeutischtechnischen Hilfsstoffen entsprechend der gewünschten Applikationsart mit einer zur Anwendung geeigneten Dosierung erfolgt in üblicher Weise, insbesondere durch Vermischen (Vgl. H. Sucker et al., Pharmazeutische Technologie, Thieme-Verlag, Stuttgart, 1978).

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions etc. in Betracht.

Die therapeutisch wirksame Verbindung ist in pharmazeutischen Zubereitungen vorzugsweise in einer Konzentration von 0,01 bis 90 Gew.-% der Gesamtmischung vorhanden.

Im allgemeinen können bei oraler Verabfolgung sowohl in der Human- als auch in der Veterinärmedizin der oder die Wirkstoffe in Mengen von etwa 1,0 bis etwa 50,0, vorzugsweise 2 bis 10 mg/kg Körpergewicht pro Tag, vorzugsweise in Form mehrerer Einzelgaben zur Erzielung der gewünschten Ergebnisse verabreicht werden. Es kann jedoch erforderlich sein, von den genannten Dosierungen abzuweichen, und zwar in Abhängigkeit von der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoffe auszukommen, während in anderen Fällen die oben angeführte Wirkstoffmenge überschritten werden muß.

Die folgenden Beispiele und Vorschriften erläutern die Herstellung der neuen Verbindungen und ihre Vorprodukte.

**Beispiel 1**

a) Herstellung der Vorprodukte

2,2 g Natriumhydrid (50 %-ige Dispersion in Mineralöl) wurden in 10 ml trockenem Dimethylformamid bei 5°C mit einer Lösung von 10,4 g Triazol in 20 ml trockenem Dimethylformamid versetzt und 10 min bei Raumtemperatur gerührt. Anschließend wurden 22,3 g cis-2-Brommethyl-2-(2,4-dichlorphenyl)-4-benzoyloxymethyl-1,3-dioxolan in 100 ml trockenem Dimethylformamid zugesetzt und nach Zugabe von 1 g Kaliumiodid über Nacht bei 145°C gerührt. Die abgekühlte Reaktionslösung wurde in Wasser gegossen und mit Essigester extrahiert. Die eingedampften Extrakte wurden in Diethylether aufgenommen, die basischen Anteile mit Chlorwasserstoff-Gas ausgefällt und das überstehende Lösungsmittel abdekantiert. Der zurückbleibende Niederschlag wurde in 100 ml Dioxan aufgenommen und nach Zugabe von 100 ml 2N Natronlauge 2 h unter Rückfluß gerührt. Die Reaktionslösung wurde am Rotationsverdampfer auf das halbe Volumen eingeengt, in Wasser gegossen und mit Methylenchlorid extrahiert. Die Extrakte wurden neutral gewaschen, über Magnesiumsulfat getrocknet und filtriert. Nach Abdampfen des Lösungsmittels und Umkristallisieren des Rückstandes aus Essigester wurden 13,7 g (83 %) cis-2-(1,2,4-Triazol-1-ylmethyl)-2-(2,4-dichlor-phenyl)-4-hydroxymethyl-1,3-dioxolan erhalten, Fp. 134 bis 137°C.

Zu 3,96 g cis-2-(1,2,4-Triazol-1-ylmethyl)-2-(2,4-dichlorphenyl)-4-hydroxymethyl-1,3-dioxolan in 50 ml trockenem Methylenchlorid und 1,44 g Triethylamin wurden 1,63 g Methansulfonsäurechlorid getropft und die Reaktionslösung bei Raumtemperatur gerührt. Nach beendeter Reaktion wurde auf Wasser gegossen, mit Methylenchlorid extrahiert, die organische Phase mit Wasser gewaschen, über Magnesiumsulfat getrocknet, filtriert und das Lösungsmittel abgedampft. Der Rückstand kristallisierte aus Isopropanol und erbrachte 4,2 g (85,7 %) cis-2-(1,2,4-Triazol-1-ylmethyl)-2-(2,4-dichlorphenyl)-4-methansulfonyl-oxymethyl-1,3-dioxolan, Fp. 95,5 bis 97°C.

b) Herstellung des Endproduktes

4,2 g cis-2-(1,2,4-Triazol-1-ylmethyl)-2-(2,4-dichlorphenyl)-4-methan-sulfonyloxymethyl-1,3-dioxolan wurden mit 6,6 g Natriumazid in 50 ml trockenem Dimethylformamid 30 min bei 100°C gerührt. Die Reaktionslösung wurde auf Wasser gegossen und mit Essigester extrahiert; die Extrakte wurden mit Wasser gewaschen, über Magnesiumsulfat getrocknet und eingedampft. Der in Aceton aufgenommene sirupöse Rückstand lieferte nach Zugabe von Chlorwasserstoff 3,5 g (86,7 %) cis-2-(1,2,4-Triazol-1-ylmethyl)-2-(2,4-dichlorphenyl)-4-azidomethyl-1,3-dioxolan als Hydrochlorid, Fp. 164 bis 166°C.

**Beispiel 2**

a) Herstellung der Vorprodukte

268 g ω-Brom-2,4-dichlor-acetophenon und 121 g 2-Hydroxymethyl-2-methylpropan-1,3-diol wurden mit 2 g p-Toluolsulfonsäure in 1000 ml Toluol am Wasserabscheider unter Rückfluß gekocht. Nach beendeter Reaktion wurde die erkaltete Lösung mit Natriumcarbonat-Lösung und Wasser neutral gewaschen, über Natriumsulfat getrocknet und das Lösungsmittel abgedampft. Der ölige Rückstand wurde an einer 800 g-Kieselgel-Filtriersäule mit Hexan/Essigester (20 : 1) gereinigt; die vereinigten Produktfraktionen lieferten nach Abdampfen des Lösungsmittels 255 g (69 %) 2-Brommethyl-2-(2,4-dichlorphenyl)-5-hydroxymethy 1-5-methyl-1,3-dioxan als ölig-zähes Gemisch beider Diastereomere, das sich allmählich verfestigte (Schmelzbereich: 90 bis 100°C).

45,8 g dieses Diastereomerengemisches wurden mit 15,3 g Triethylamin in 200 ml trockenem Tetrahydrofuran nach Zugabe von 21,3 g Benzoylchlorid 2 h bei Raumtemperatur gerührt. Der Niederschlag wurde abfiltriert, das Lösungsmittel weitgehend abgedampft, der Rückstand in Essigester aufgenommen, mit Wasser gewaschen, über Natriumsulfat getrocknet und die Lösung nach Filtrieren eingedampft. Der Rückstand lieferte nach Umkristallisieren aus Ethanol 37 g (63,4 %) 2-Brom-methyl-2-(2,4-dichlorphenyl)-5-benzoyloxymethyl-5-methyl-1,3-dioxan (Isomer A). Fp. 139 bis 142°C.

8,5 g 2-Brommethyl-2-(2,4-dichlorphenyl)-5-benzoyloxymethyl-5-methyl-1,3-dioxan (Isomer A) und 4,9 g Imidazol wurden mit 0,3 g Kaliumiodid in 30 ml trockenem Dimethylformamid unter Stickstoff zum Rückfluß erhitzt.

Nach beendeter Reaktion wurde die erkaltete Lösung auf Wasser gegossen, mit Essigester extrahiert, die eingedampften Extrakte in 50 ml Dioxan aufgenommen und mit 10 ml 2N Natronlauge 1,5 h unter Rückfluß gerührt. Die erkaltete Lösung wurde auf Wasser gegossen und mit Methylenchlorid extrahiert. Trocknen der Extrakte über Natriumsulfat und Abdampfen des Lösungsmittels lieferte einen Rückstand, aus dem nach Umkristallisieren mit Diethylether 4,7 g (73,5 %) 2-(Imidazol-1-ylmethyl)-2-(2,4-dichlorphenyl)-5-hydroxymethyl-5-methyl-1,3-dioxan (Isomer A) erhalten wurden, Fp. 174 bis 175°C.

b) Herstellung des Endproduktes

1,78 g 2-(Imidazol-1-ylmethyl)-2-(2,4-dichlorphenyl)-5-hydroxymethyl-5-methyl-1,3-dioxan (Isomer A) und 0,6 g Triethylamin wurden in 30 ml trockenem Methylenchlorid mit 1,1 g p-Toluolsulfonsäurechlorid versetzt. Nach 2 h Rühren bei Raumtemperatur wurde auf Wasser gegossen, mit Methylenchlorid extrahiert, die organische Phase mit Wasser gewaschen, über Magnesiumsulfat getrocknet, filtriert und eingedampft.

Der Rückstand wurde in 20 ml trockenem Dimethylformamid aufgenommen und nach Zugabe von 3,3 g

Natriumazid 2 h bei 100° C gerührt. Die erkaltete Lösung wurde auf Wasser gegossen, mit Essigester extrahiert, die organische Phase mit Wasser nachgewaschen, über Natriumsulfat getrocknet, filtriert und zur Trockne eingedampft. Der Rückstand lieferte nach Umkristallisieren aus Diisopropylether 1,3 g (68 %) 2-(Imidazol-1-ylmethyl)-2-(2,4-dichlorphenyl)-5-azidomethyl-5-methyl-1,3-dioxan, Fp. 132 bis 133° C.

Entsprechend Beispiel 1 und 2 wurden bzw. können die in der Tabelle 2 aufgeführten Verbindungen hergestellt werden.

**Tabelle 2**

| Beispiel | Z | R | $R^1$ | m | Salz | Fp [°C] |
|---|---|---|---|---|---|---|
| 3 | N | $C(CH_3)_3$ | $CH_3$ | 1 | | |
| 4 | CH | $2{,}4\text{-}Cl_2\text{-}C_6H_3$ | $C_2H_5$ | 1 | | |
| 5 | N | $C(CH_3)_3$ | H | 0 | HCl | 172-173 |
| 6 | N | $2{,}4\text{-}Cl_2\text{-}C_6H_3$ | $n\text{-}C_3H_7$ | 1 | | |
| 7 | CH | $2{,}4\text{-}Cl_2\text{-}Cl_2\text{-}C_6H_3$ | H | 0 | | 85 |
| 8 | CH | $2{,}4\text{-}Cl_2\text{-}C_6H_3$ | $CH(CH_3)_2$ | 1 | | |
| 9 | CH | $C(CH_3)_3$ | $CH_3$ | 1 | | |
| 10 | CH | $C(CH_3)_3$ | $n\text{-}C_3H_7$ | 1 | | |
| 11 | CH | $C(CH_3)_3$ | H | 0 | | |
| 12 | N | $4\text{-}Cl\text{-}C_6H_4$ | $CH_3$ | 1 | | |
| 13 | N | $2{,}4\text{-}Cl_2\text{-}C_6H_3$ | $C_2H_5$ | 1 | | |
| 14 | N | $4\text{-}F\text{-}C_6H_4$ | H | 0 | | |
| 15 | CH | $4\text{-}F\text{-}C_6H_4$ | H | 0 | | |

Beispiele für pharmazeutische Zubereitungen:

**BEISPIEL A**
Tablette mit 250 mg Wirkstoff

Zusammensetzung für 1000 Tabletten:

| | |
|---|---|
| Wirkstoff des Beispiels Nr. 7 | 250 g |
| Kartoffelstärke | 100 g |
| Milchzucker | 50 g |
| Gelatinelösung 4 % | 45 g |
| Talkum | 10 g |

**Herstellung:**

Der fein gepulverte Wirkstoff, Kartoffelstärke und Milchzucker werden gemischt. Die Mischung wird mit ca. 45 g 4 % Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpreßt. Die Tabletten werden in dicht schließende Behälter aus Polypropylen gefüllt.

**BEISPIEL B**
Creme aus 1 % Wirkstoff

| | |
|---|---|
| Wirkstoff des Beispiels Nr. 7 | 1,0 g |
| Glycerinmonostearat | 10,0 g |
| Cetylalkohol | 4,0 g |
| Polyethylenglykol-400-stearat | 10,0 g |
| Polyethylenglykol-sorbitanmonostearat | 10,0 g |
| Propylenglykol | 6,0 g |
| p-Hydroxybenzoesäuremethylester | 0,2 g |
| Entmineralisiertes Wasser | ad 100,0 g |

**Herstellung:**

Der feinst gepulverte Wirkstoff wird mit Propylenglykol suspendiert und die Suspension in die auf 65°C erwärmte Schmelze aus Glycerinmonostearat, Cetylalkohol, Polyethylenglykol-400-stearat und Polyethylenglykolsorbitanmonostearat gerührt. In diese Mischung wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

**BEISPIEL C**
Puder mit 1 % Wirkstoff

| | |
|---|---|
| Wirkstoff des Beispiels Nr. 7 | 1,0 g |
| Zinkoxid | 10,0 g |
| Magnesiumoxid | 10,0 g |
| Hochdisperses Siliciumdioxid | 2,5 g |
| Magnesiumstearat | 1,0 g |
| Talkum | 75,5 g |

**Herstellung:**

Der Wirkstoff wird auf einer Luftstrahlmühle mikronisiert und mit den anderen Bestandteilen homogen vermischt. Die Mischung wird durch ein Sieb (Maschenweite Nr. 7) geschlagen und in Polyethylenbehälter mit Streueinsatz abgefüllt.

**Patentansprüche** für die Vertragsstaaten BE CH DE FR GB IT LI NL

1. Azolylmethylcycloacetale der Formel I

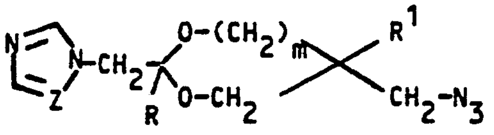 (I),

in der
R einen Phenylrest, der durch Halogen substituiert sein kann, oder eine $C_{1-6}$-Alkylgruppe,
$R^1$ Wasserstoff oder einen $C_{1-3}$-Alkylrest,
Z CH oder N und
m die Zahl 0 oder 1
bedeuten, sowie deren physiologisch verträgliche Säureadditionssalze.

2. Azolylmethylcycloacetale der Formel I gemäß Anspruch 1, dadurch gekennzeichnet, daß
R einen tert.-Butylrest oder einen durch Halogen substituierten Phenylrest,
$R^1$ Wasserstoff oder eine Methylgruppe,
Z CH oder N und
m 0 oder 1
bedeuten sowie deren physiologisch verträgliche Säureadditionssalze.

3. 2-(2,4-Dichlorphenyl)-2-(1H-imidazol-1-ylmethyl)-1,3-dioxolan-4-yl-methylazid sowie seine physiologisch verträglichen Säureadditionssalze.

4. 2-(2,4-Dichlorphenyl)-2-(1H-imidazol-1-ylmethyl)-5-methyl-1,3-di-oxolan-5-ylmethylazid sowie seine physiologisch verträglichen Säureadditionssalze.

5. 2-(2,4-Dichlorphenyl)-2-(1H-1,2,4-triazol-1-ylmethyl)-1,3-dioxolan-4-ylmethylazid sowie seine physiologisch verträglichen Säureadditionssalze.

6. Verfahren zur Herstellung von Azolylmethylcycloacetalen der Formel I gemäß Anspruch 1, dadurch gekennzeichnet, daß man

a) ein Cycloacetal der Formel II

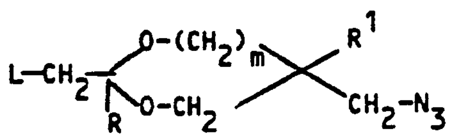 (II),

in der R, $R^1$ und m die oben angegebene Bedeutung haben und L eine nucleophil substituierbare Abgangsgruppe darstellt, mit einer Verbindung der Formel III

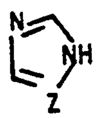 (III),

in der Z die oben angegebene Bedeutung hat, oder

b) ein Cycloacetal der Formel IV

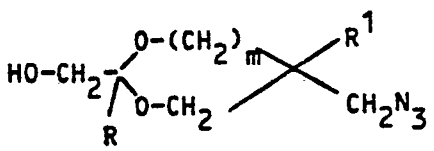 (IV),

worin R, $R^1$ und m die oben angegebene Bedeutung besitzen, mit einer Verbindung der Formel V

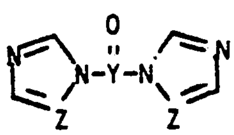 (V),

in welcher Z die oben angegebene Bedeutung hat und Y ein Kohlenstoff- oder Schwefelatom bedeutet, oder

c) eine Verbindung der Formel VI

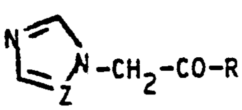 (VI),

in der Z und R die oben angegebene Bedeutung haben, mit einer Verbindung der Formel VII

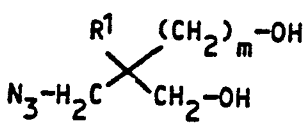 (VII),

in der $R^1$ und m die oben angegebene Bedeutung haben, oder

d) eine Verbindung der Formel VIII

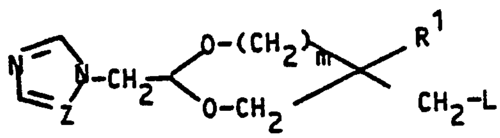 (VIII),

in der Z, R, $R^1$ und m die oben angegebene Bedeutung haben und L eine nucleophil substituierbare Abgangsgruppe darstellt, mit einem Alkalimetallazid

umsetzt und die so erhaltenen Verbindungen gegebenenfalls in ihre physiologisch verträglichen Säureadditionssalze überführt.

7. Verbindung der Formel I gemäß Amsprüchen 1 bis 5 zur Verwendung bei der Bekämpfung von Krankheiten.

8. Verwendung der Verbindungen der Formel I gemäß Anspruch 1 zur Herstellung eines Arzmeimittels zur Bekämpfung von Mykosen.

**Patentansprüche** für den Vertragsstaat AT

Verfahren zu Herstellung von

1. Azolylmethylcycloacetalen der Formel I

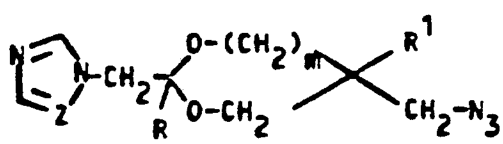 (I),

in der

R einen Phenylrest, der durch Halogen substiturert sein kann, oder eine $C_{1-6}$-Alkylgruppe,

$R^1$ Wasserstoff oder einen $C_{1-3}$-Alkylrest,

Z CH oder H und

m die Zahl 0 oder 1

bedeuten, sowie deren physiologisch verträglichen Säureadditionssalzen,

dadurch gekennzeichnet, daß man

a) ein Cycloacetel der Formel II

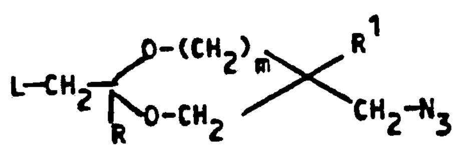 (II),

in der R, $R^1$ und m die oben angegebene Bedeutung haben und L eine nucleophil substituierbare Abgangsgruppe darstellt, mit einer Verbindung der Formel III

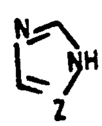 (III),

in der Z die oben angegebene Bedeutung bat, oder

b) ein Cycloacetal der Formel IV

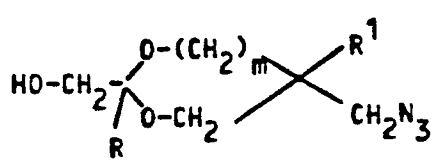 (IV),

worin R, $R^1$ und m die oben angegebene Bedeutung besitzen, mit einer Verbindung der Formel V

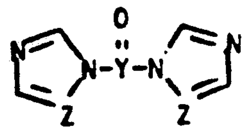

(V),

in welcher Z die oben angegebene Bedeutung hat und Y ein Kohlenstoff- oder Schwefelatom bedeutet, oder
c) eine Verbindung der Formel VI

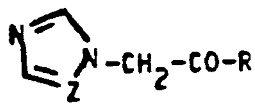

(VI),

in der Z und R die oben angegebene Bedeutung haben, mit einer Verbindung der Formel VII

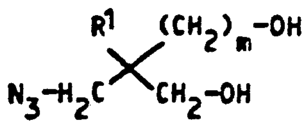

(VII),

in der $R^1$ und m die oben angegebene Bedeutung haben, oder
d) eine Verbindung der Formel VIII

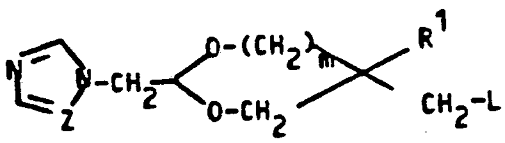

(VIII),

in der Z, R, $R^1$ und m die oben angegebene Bedeutung haben und L eine nucleophil substituierbare Abgangsgruppe darstellt, mit einem Alkalimetallazid
umsetzt und die so erhaltenen Verbindungen gegebenenfalls in ihre physiologisch verträglichen Säureadditionssalze überführt.

2. Verfahren gemäß Anspruch 1, dadurch gekennzeichnet, daß man Verbindungen der Formel I gemäß Anspruch 1 oder deren Salze und physiologisch verträglichen Säuren erstellt, worin
R einen tert.-Butylrest oder einen durch Halogen substituierten Phenylrest,
$R^1$ Wasserstoff oder eine Methylgruppe,
Z CH oder N und
m 0 oder 1
bedeuten.

3. Verfahren gemäß Anspruch 1, dadurch gekennzeichnet, daß man 2-(2,4-Dichlorphenyl)-2-(1H-imidazol-1-ylmethyl)-1,3-dioxolan-4-yl-methylazid oder dessen physiologisch verträglichen Säureadditionssalze herstellt.

4. Verfahren gemäß Anspruch 1, dadurch gekennzeichnet, daß man 2-(2,4-Dichlorphenyl)-2-(1H-imidazol-1-ylmethyl)-5-methyl-1,3-di-oxolan-5-ylmethylazid oder dessen physiologisch verträglichen Säureadditionssalze herstellt.

5. Verfahren gemäß Anspruch 1, dadurch gekennzeichnet, daß man 2-(2,4-Dichlorphenyl)-2-(1H-1,2,C-triazol-1-ylmethyl)-1,3-dioxolan-4-ylmethylazid oder dessen physiologisch verträglichen Säureadditionssalze herstellt.

**Claims** for the Contracting states BE, CH, DE, FR, GB, IT, LI, NL

1. An azolylmethylcycloacetal of the formula I

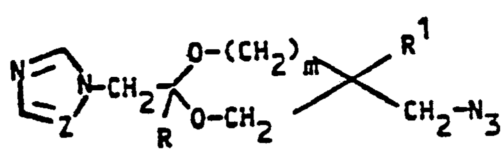

(I),

where
R is phenyl which can be substituted by halogen, or is $C_1$-$C_6$-alkyl,
$R^1$ is hydrogen or $C_1$-$C_3$-alkyl,
Z is CH or N, and

m is 0 or 1,

and its physiologically tolerated addition salts with acids.

2. An azolylmethylcycloacetal of the formula I as claimed in claim 1, wherein

R is tert.-butyl or halogen-substituted phenyl,

R is hydrogen or methyl,

Z is CH or N, and

m is 0 or 1,

and its physiologically tolerated addition salts with acids.

3. 2-(2,4-Dichlorophenyl)-2-(1H-imidazol-1-ylmethyl)-1,3-di-oxolan-4-ylmethyl azide and its physiologically tolerated addition salts with acids.

4. 2-(2,4-Dichlorophenyl)-2-(1H-imidazol-1-ylmethyl)-5-methyl-1,3-dioxolan-5-ylmethyl azide and its physiologically tolerated addition salts with acids.

5. 2-(2,4-Dichlorophenyl)-2-(1H-1,2,4-triazol-1-ylmethyl)-1,3-dioxolan-4-ylmethyl azide and its physiologically tolerated addition salts with acids.

6. A process for the preparation of an azolylmethylcycloacetal of the formula I as claimed in claim 1, wherein

a) a cycloacetal of the formula II

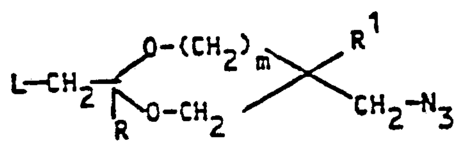

(II),

where R, $R^1$ and m have the above meanings and L is a leaving group which can undergo nucleophilic substitution, is reacted with a compound of the formula III

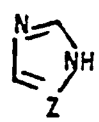

(III),

where Z has the above meanings, or

b) a cycloacetal of the formula IV

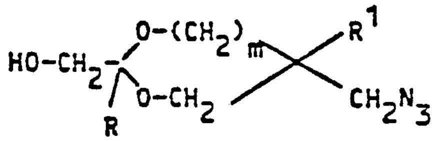

(IV),

where R, $R^1$ and m have the above meanings, is reacted with a compound of the formula V

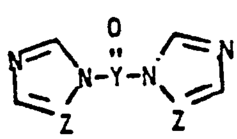

(V),

where Z has the above meanings and Y is a carbon or sulfur atom, or

c) a compound of the formula VI

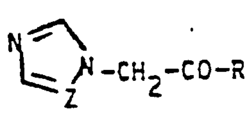

(VI),

where Z and R have the above meanings, is reacted with a compound of the formula VII

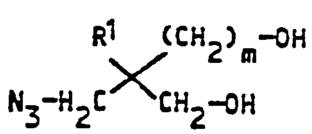

(VII),

where $R^1$ and m have the above meanings, or

d) a compound of the formula VIII

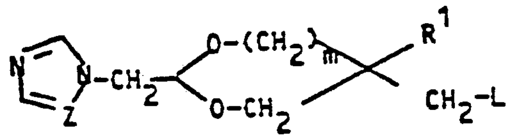 (VIII),

where Z, R, $R^1$ and m have the above meanings and L is a leaving group which can undergo nucleophilic substitution, is reacted with an alkali metal azide,

and the resulting compound is, if desired, converted to its physiologically tolerated addition salts with acids.

7. A compound of the formula I as claimed in claims 1 to 5 for use in the treatment of disorders.

8. Use of a compound of the formula I as claimed in claim 1 for the preparation of a drug for treating mycoses.

**Claims** for the Contracling State AT

1. A process for the preparation of an azolylmethylcycloacetal of the formula I

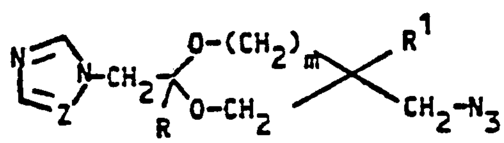 (I),

where

R is phenyl which can be substituted by halogen, or is $C_1$-$C_6$-alkyl,

$R^1$ is hydrogen or $C_1$-$C_3$-alkyl,

Z is CH or N, and

m is 0 or 1,

or its physiologically tolerated addition salts with acids, wherein

a) a cycloacetal of the formula II

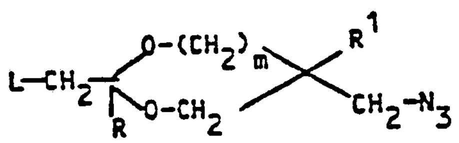 (II),

where R, $R^1$ and m have the above meanings and L is a leaving group which can undergo nucleophilic substitution, is reacted with a compound of the formula II

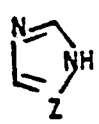 (III),

where Z has above meanings, or

b) a cycloacetal of the formula IV

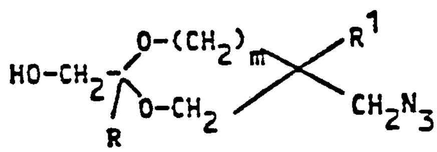 (IV),

where R, $R^1$ and m have the above meanings, is reacted with a compound of the formula V

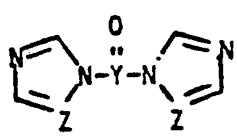 (V),

where Z has the above meanings and Y is a carbon or sulfur atom, or

c) a compound of the formula VI

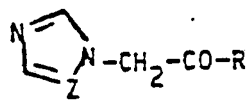

(VI),

where Z and R have above meanings, is reacted with a compound of the formula VII

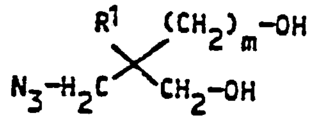

(VII),

where $R^1$ and m have the above meanings, or

d) a compound of the formula VIII

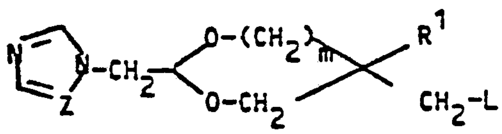

(VIII),

where Z, R, $R^1$ and m have the above meanings and L is a leaving group which can undergo nucleophilic substitution, is reacted with an alkali metal azide,

and the resulting compound is, if desired, converted to its physiologically tolerated addition salts with acids.

2. A process as claimed in claim 1, werein a compound of the formula I as claimed in claim 1, where

R is tert.-butyl or halogen-substituted phenyl,
$R^1$ is hydrogen or methyl,
Z is CH or N, and
m is 0 or 1,

or its salts with physiologically tolerated acids are prepared.

3. A process as claimed in claim 1, wherein 2-(2,4-dichloro-phenyl)-2-(1H-imidazol-1-ylmethyl)-1,3-dioxolan-4-ylmethyl azide or its physiologically tolerated addition salts with acids are prepared.

4. A process as claimed in claim I, wherein 2-(2,4-dichloro-phenyl)-2-(1H-imidazol-1-ylmethyl)-5-methyl-1,3-dioxolan-5-ylmethyl azide or its physiologically tolerated addition salts with acids are prepared.

5. A process as claimed in claim 1, wherein 2-(2,4-dichloro-phenyl)-2-(1H-1,2,4-triazol-1-ylmethyl)-1,3-dioxolan-4-ylmethyl azide or its physiologically tolerated addition salts with acids are prepared.

**Revendications** pour les Etats contractants BE, CH, DE, FR, GB, IT, LI, NL

1. Azolylméthylcycloacétals de la formule I

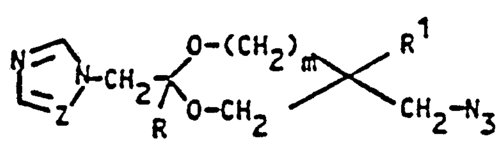

(I),

dans laquelle

R représente une groupe phényle pouvant être halosubstitué ou un radical alkyle en $C_1$ à $C_6$,
$R^1$ désigne un atome d'hydrogène ou un radical alkyle en $C_1$ à $C_3$,
Z = CH ou N et
m vaut 0 ou 1,
et leurs sels d'addition d'acides physiologiquement acceptables.

2. Azolylméthylcycloacétals de la formule I selon la revendication 1, caractérisés en ce que
R désigne un radical butyle tertiaire ou un groupe phényle halo-substitué,
$R^1$ = atome d'hydrogène ou radical méthyle,
Z = CH ou N et
m =o ou 1,
ainsi que les sels d'addition d'acides physiologiquement acceptables de ces composés.

3. Le 2- (2, 4-diclorophényl) -2-(1 H-imidazol-1-ylméthyl)-1,3-dioxolan-4-yl-méthylazide et ses sels d' addition d'acides physiologiquement acceptables.

4. Le 2- (2, 4-dichlorophényl) -2- (1 H-imidazol-1 -ylméthyl)-5-méthyl-1, 3-dioxolan-5-yl-méthylazide et ses sels d'addition d'acides physiologiqument acceptables.

5. Le 2-(2,4-dichlorophényl)-2-(1H-1,2,4-triazol-1-ylméthyl)-1, 3-dioxolan-4-yl-méthylazide et ses sels

d'addition d'acides physiologiquement acceptables.

6. Procédé de préparation d'azolylméthylcycloacétals de la formule I selon la revendication 1, caractérisé en ce que l'on fait réagir:

a) un cycloacétal de la formule II

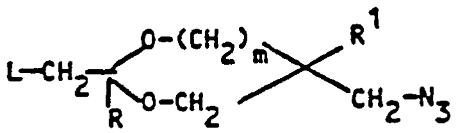

(II),

dans laquelle R, $R^1$ et m possèdent la signification définie ci-dessus et L représente un groupe clivable, pouvant être substitué par voie nucléophile, avec un composé de la formule III

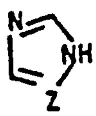

(III),

dans laquelle Z possède la signification définie, ou

b) un cycloacétal de la formule IV

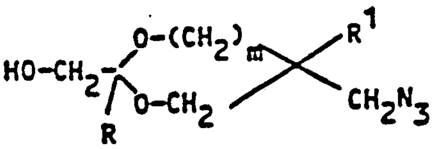

(IV),

dans laquelle R, $R^1$ et m possèdent la signification définie plus haut,
avec un composé de la formule V

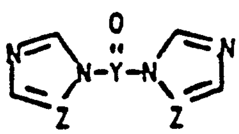

(V),

dans laquelle Z possède la signification définie et Y désigne un atome de carbone ou de soufre, ou

c) un composé de la formule VI

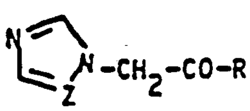

(VI),

dans laquelle Z et R possèdent la signification définie plus haut, avec un composé de la formule VII

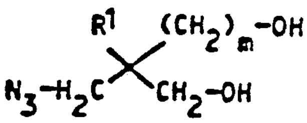

(VII),

dans laquelle $R^1$ et m possèdent la signification définie, ou

d) un composé de la formule VIII

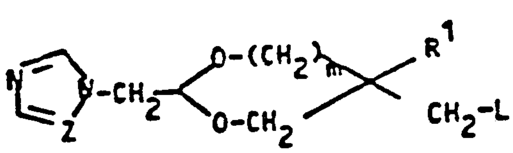

(VIII),

dans laquelle Z, R, $R^1$ et m possèdent la signification définie plus haut et L désigne un groupe clivable, pouvant être substitué par voie nucléophile, avec un azide d'un métal alcalin,

les composés obtenus pouvant éventuellement être transformés en leurs sels d'addition d'acides physiologiquement acceptables.

7. Composé de la formule I selon les revendications 1 à 5, utilisé pour le traitement de maladies.

8. Utilisation des composés de la formule I selon la revendication 1 pour la préparation de médicaments pour le traitement de mycoses.

**Revendications** pour l'Etat contractant AT

1. Procédé de préparation d'azolylméthylcycloacétals de la formule I

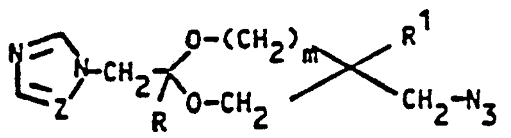

(I),

dans laquelle

R représente une groupe phényle pouvant être halosubstitué ou un radical alkyle en $C_1$ à $C_6$,
$R^1$ désigne un atome d'hydrogène ou un radical alkyle en $C_1$ à $C_3$,
Z = CH ou N et
m vaut 0 ou 1,
et de leurs sels d'addition d'acides physiologiquement acceptables,
caractérisé en ce que l'on fait réagir:
a) un cycloacétal de la formule II

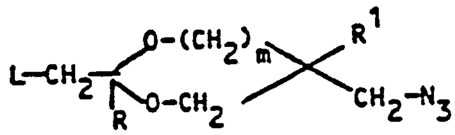

(II),

dans laquelle R, $R^1$ et m possèdent la signification définie ci-dessus et L représente un groupe clivable, pouvant être substitué par voie nucléophile, avec un composé de la formule III

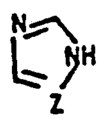

(III),

dans laquelle Z possède la signification définie, ou
b) un cycloacétal de la formule IV

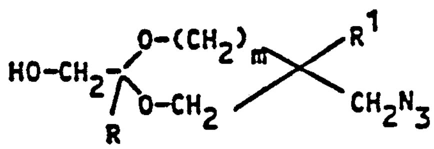

(IV),

dans laquelle R, $R^1$ et m possèdent la signification définie plus haut, avec un composé de la formule V

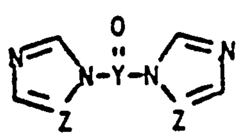

(V),

dans laquelle Z possède la signification définie et Y désigne un atome de carbone ou de soufre, ou
c) un composé de la formule VI

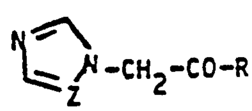

(VI),

dans laquelle Z et R possèdent la signification définie plus haut, avec un composé de la formule VII

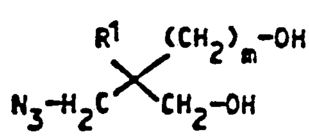

(VII),

dans laquelle $R^1$ et m possèdent la signification définie, ou
d) un composé de la formule VIII

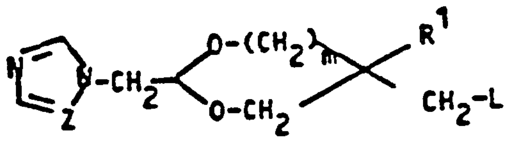

(VIII),

dans laquelle Z, R, $R^1$ et m possèdent la signification définie plus haut et L désigne un groupe clivable, pouvant être substitué par voie nucléophile, avec un azide d'un métal alcalin,

les composés obtenus pouvant éventuellement être transformés en leurs sels d'addition d'acides physiologiquement acceptables.

2. Procédé suivant la revendication 1, caractérisé en ce que l'on prépare des composés de la formule I selon la revendication 1, pour lesquels

R désigne un radical butyle tertiaire ou un groupe phényle halo-substitué,

$R^1$ = atome d'hydrogène ou radical méthyle,

Z = CH ou N et

m = 0 ou 1,

ou des sels d'acides physiologiquement acceptables de ceux-ci.

3. Procédé suivant la revendication 1, caractérisé en ce que l'on prépare le 2- (2, 4-dichlorophényl) -2- (1H-imidazol-1 -ylméthyl) -1, 3-dioxolan-4-yl-méthylazide ou ses sels d'addition d'acides physiologiquement acceptables.

4. Procédé suivant la revendication 1, caractérisé en ce que l'on prépare le 2- (2, 4-dichlorophényl) -2- (1H-imidazol-1 -ylméthyl) -5-méthyl-1, 3-dioxolan-5-yl-méthyl-azide ou ses sels d'addition d'acides physiologiquement acceptables.

5. Procédé suivant la revendication 1, caractérisé en ce que l'on prépare le 2- (2, 4-dichlorophényl)-2- (1H-1, 2, 4-triazol-1 -ylméthyl) -1, 3-dioxolan-4-yl-méthylazide ou ses sels d'addition d'acides physiologiquement acceptables.